(19) Europäisches Patentamt
European Patent Office
Office européen des brevets

(11) Numéro de publication: **0 350 384 B1**

(12) # FASCICULE DE BREVET EUROPEEN

(45) Date de publication de fascicule du brevet: **25.05.94**

(51) Int. Cl.[5]: **C07D 471/04**, //(C07D471/04, 221:00,209:00)

(21) Numéro de dépôt: **89401904.1**

(22) Date de dépôt: **03.07.89**

Le dossier contient des informations techniques présentées postérieurement au dépôt de la demande et ne figurant pas dans le présent fascicule.

(54) **Dérivés de sulfonyl indolizine, leur procédé de préparation et leur utilisation comme intermédiaires de synthèse.**

(30) Priorité: **04.07.88 FR 8809023**

(43) Date de publication de la demande:
**10.01.90 Bulletin 90/02**

(45) Mention de la délivrance du brevet:
**25.05.94 Bulletin 94/21**

(84) Etats contractants désignés:
**AT BE CH DE ES FR GB GR IT LI LU NL SE**

(56) Documents cités:
**EP-A- 302 792**
**EP-A- 302 793**
**EP-A- 0 235 111**

(73) Titulaire: **ELF SANOFI**
**32-34, rue Marbeuf**
**F-75008 Paris(FR)**

(72) Inventeur: **Gubin, Jean**
**avenue des Citronniers 24,**
**B-1020 Bruxelles 2(BE)**
Inventeur: **Lucchetti, Jean**
**Rue des Combattants 23A**
**B-5860 Chastre(BE)**

(74) Mandataire: **Le Guen, Gérard et al**
**CABINET LAVOIX**
**2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte, d'une manière générale, à la préparation de dérivés d'aminoalkoxyphényl sulfonyl-3-indolizine et plus particulièrement à de nouveaux intermédiaires de synthèse et à leur procédé de préparation.

Plus précisément, l'invention concerne des dérivés de sulfonyl-3 indolizine de formule générale :

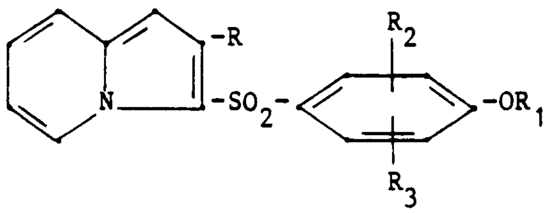

I

dans laquelle : R représente l'hydrogène, un radical alkyle, linéaire ou ramifié en $C_1$-$C_8$, un radical cycloalkyle en $C_3$-$C_6$ ou un groupement phényle non substitué ou substitué par un ou plusieurs substituants, identiques ou différents, choisis parmi des atomes d'halogène, par exemple fluor, chlore, brome et parmi des groupements alkyle en $C_1$-$C_4$,alkoxy en $C_1$-$C_4$ ou nitro.

$R_1$ représente un radical protecteur d'un groupement hydroxyle à savoir un groupement méthyle, benzyle, alkylsulfonyle en $C_1$-$C_4$ par exemple méthanesulfonyle,ou arylsulfonyle en $C_6$-$C_{10}$ par exemple benzènesulfonyle ou p-toluènesulfonyle.

$R_2$ et $R_3$, identiques ou différents, représentent chacun l'hydrogène, le radical méthyle ou éthyle ou un atome d'halogène.

Par "radical alkyle linéaire ou ramifié en $C_1$-$C_8$ "on désigne notamment les radicaux méthyle, éthyle, n-propyle, isopropyle, n-butyle, isobutyle, tertiobutyle, n-pentyle, néopentyle, n-hexyle, n-heptyle ou n-octyle.

De même, par "radical cycloalkyle en $C_3$-$C_6$", on entend notamment les radicaux cyclopropyle ou cyclohexyle.

Ainsi, en tenant compte des valeurs données ci-dessus R peut représenter notamment un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromo-phényle, di-fluoro-, di-chloro-ou di-bromo-phényle, mono-méthyl- ou di-méthyl-phényle, mono-méthoxy- ou di-méthoxy-phényle ou un radical méthylphényle substitué par un atome d'halogène.

Parmi les composés de formule I, ceux dans laquelle R représente un radical isopropyle ou cyclopropyle constituent des composés préférés.

Contrairement à ce que l'on pouvait attendre on est parvenu à préparer les composés de formule I d'une manière particulièrement simple.

La présente invention a donc également pour objet un procédé inattendu de préparation des composés de formule I.

Les dérivés d'indolizine de formule I sont particulièrement utiles comme produits intermédiaires notamment pour la préparation des dérivés d'aminoalkoxyphénylsulfonyl-3 indolizine de formule générale :

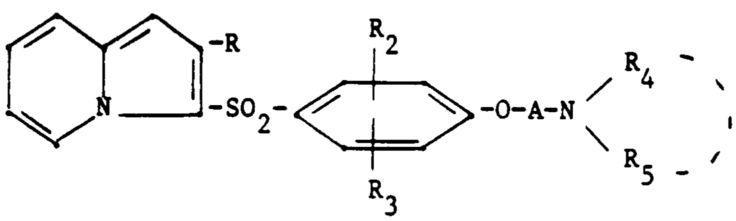

Ia

ainsi que de leurs sels pharmaceutiquement acceptables, dans laquelle R, $R_2$ et $R_3$ ont la même signification que précédemment,

A représente un radical alkylène, linéaire ou ramifié en $C_2$-$C_5$ ou le radical hydroxy-2 propylène dans lequel l'hydroxy est éventuellement substitué par un radical alkyle en $C_1$-$C_4$,

$R_4$ représente un radical alkyle linéaire ou ramifié en $C_1$-$C_8$ ou un radical de formule :

-Alk-$R_6$

dans laquelle Alk représente une liaison simple ou un radical alkylène, linéaire ou ramifié, en $C_1$-$C_5$ et $R_6$ représente un radical pyridyle, phényle, méthylènedioxy-2,3 phényle, méthylènedioxy-3,4 phényle ou un groupement phényle substitué par un ou plusieurs substituants identiques ou différents choisis parmi des atomes d'halogène, des groupements alkyle en $C_1$-$C_4$ ou des groupements alkoxy en $C_1$-$C_4$,

$R_5$ représente l'hydrogène ou un radical alkyle en $C_1$-$C_8$ ou $R_4$ et $R_5$, lorsqu'ils sont pris ensemble représentent un radical alkylène ou alkénylène en $C_3$-$C_6$ éventuellement substitué par un radical phényle ou éventuellement interrompu par -O-, -N= ou -N-$R_7$ , $R_7$ représentant l'hydrogène, un radical alkyle en $C_1$-$C_4$ ou un radical phényle.

Ces composés de formule Ia ont été revendiqués dans les demandes de brevets européens Nos. 302 792 et 302 793 (publiées postérieurement à la date de dépôt de la présente demande) car ils se sont révélés doués de remarquables propriétés pharmacologiques notamment des propriétés inhibitrices de la translocation calcique ainsi que des propriétés bradycardisantes, hypotensives et antiadrénergiques. Ces propriétés,prises dans leur ensemble, rendent les composés en question particulièrement utiles dans le traitement de certains syndromes pathologiques du système cardiovasculaire en particulier dans le traitement de l'angine de poitrine, de l'hypertension, de l'arythmie, de l'insuffisance circulatoire cérébrale.

De ce point de vue, l'un des composés de formule Ia les plus représentatifs est l'isopropyl-2[{[ N-méthyl-N-(diméthoxy-3,4 $\beta$-phénéthyl)amino]-3 propyloxy}-4 phénylsulfonyl]-3 indolizine et ses sels pharmaceutiquement acceptables.

On a peu étudié jusqu'à présent l'oxydation de sulfures indoliziniques en sulfones correspondantes probablement en raison de la dégradation, bien connue, du cycle indolizine sous l'effet d'agents oxydants. On a décrit, par exemple dans "Heterocyclic Systems with Bridgehead Nitrogen Atoms", Part one, Intersc. Publ., New York, 1961 p. 263, l'ouverture du cycle indolizine en acide picolinique -N-oxyde et homologues supérieurs, sous l'action du peroxyde d'hydrogène dans l'acide acétique.

Cependant, on a mentionné dans la demande de brevet européen No. 235.111, l'oxydation de dérivés d'(aminoalkoxy-4 phénylthio)-1 indolizine en dérivés sulfoxydes correspondants, au moyen de périodate de sodium, de permanganate de potassium ou d'acide chloro-3 perbenzoïque, la réaction ayant lieu dans l'eau ou le chlorure de méthylène. On a également signalé qu'au cours de cette oxydation, le dérivé sulfonyle correspondant peut être éventuellement formé sans qu'aucun exemple n'illustre toutefois cette affirmation.

Dans le cadre de l'élaboration de la présente invention, on a étudié le comportement d'une alkyl-2 p-méthoxyphénylthio-3 indolizine en l'occurrence le dérivé isopropyl-2, sous l'effet de différents agents d'oxydation.

Les résultats obtenus ont montré que l'emploi du couple périodate de sodium/permanganate de potassium dans un mélange acétone/eau n'a pas permis d'obtenir la sulfone en question.

En vue d'en diminuer le pouvoir oxydant, on a également utilisé le permanganate de potassium en présence d'un catalyseur de transfert de phase. Des essais effectués en milieu dichlorométhane avec ou sans ajout d'acide acétique se sont également soldés par des échecs.

On a également enregistré des résultats négatifs lors de l'utilisation de perborate de sodium en milieu acide ou basique ou de peroxyde d'hydrogène en milieu basique. Enfin l'emploi d'oxone (mélange de sulfate, hydrogénosulfate et hydrogénopersulfate de potassium)en milieu méthanol/eau n'a permis de déceler la sulfone recherchée qu'à l'état de traces.

On a maintenant trouvé, de manière surprenante, qu'il est possible d'effectuer, en ayant des rendements importants, l'oxydation de phénylthio-3 indolizines au moyen d'acide chloro-3 perbenzoïque en provoquant la formation préférentielle de la sulfone correspondante au détriment du dérivé sulfoxyde correspondant ou d'autres produits secondaires.

Ainsi, le procédé de l'invention pour la préparation des dérivés d'indolizine de formule I consiste à oxyder, à température ambiante, un dérivé phénylthio-3 indolizine de formule générale :

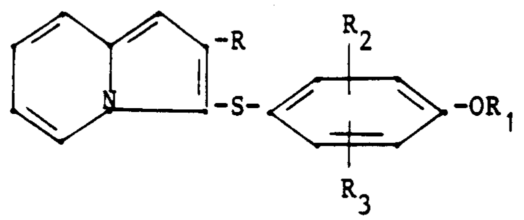

II

dans laquelle R, $R_1$, $R_2$ et $R_3$ ont la même signification que précédemment, au moyen d'acide chloro-3 perbenzoïque, en présence d'un agent basique généralement un carbonate de métal alcalin tel que le carbonate de sodium ou de potassium, et dans un alcool inférieur en $C_1$-$C_4$ comme solvant, de préférence le méthanol.

Généralement, on utilise de 1 à 5 équivalents, de préférence 4 équivalents d'acide chloro-3 perbenzoïque par équivalent de dérivé phénylthio-3 indolizine de formule II et de 2 à 4 équivalents d'agent basique, de préférence environ 2,5 équivalents par équivalent d'acide chloro-3 perbenzoïque.

Dans le cas particulier de la préparation de l'isopropyl-2 p-méthoxyphénylsulfonyl-3 indolizine, le procédé de l'invention permet d'obtenir des rendements de l'ordre de 50 à 55%.

Les composés de formule II peuvent être obtenus en faisant réagir, au reflux, et dans un solvant approprié tel qu'un alcool par exemple l'éthanol, une indolizine présentant en position 2 un substituant R (dite ci-après R-2 indolizine) avec un disulfure de formule générale :

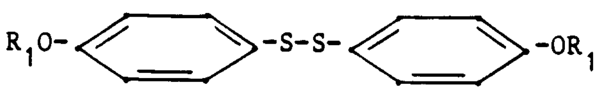

III

dans laquelle $R_1$ a la même signification que précédemment.

Les composés de formule III sont des produits connus ayant été publiés dans Bull. Soc. Chim. Fr. 33 p. 837 ou pouvant être préparés par la méthode y décrite.

De même, les R-2 indolizines sont des composés connus ayant été publiés dans le brevet français No. 2.341.578.

Comme indiqué précédemment, on a effectué une série d'essais préliminaires en vue de préparer l'isopropyl-2 p-méthoxyphénylsulfonyl-3 indolizine au départ d'isopropyl-2 p-méthoxyphénylthio-3 indolizine en utilisant différents agents d'oxydation et différents milieux réactionnels.

A cet effet, on a utilisé le procédé suivant :

On agite, à température ambiante, une solution d'un équivalent d'isopropyl-2 p-méthoxyphénylthio-3 indolizine en présence d'un agent oxydant en faisant varier :

- le milieu réactionnel
- la quantité d'agent d'oxydation

et on chauffe éventuellement le milieu au reflux puis on sépare, par chromatographie sur couche mince, les composés formés (solvant : acétate d'éthyle/hexane 3/7).

On a obtenu les résultats suivants comparativement au procédé de l'invention :

1) Essais préliminaires

| Agent d'oxydation | | Milieux réactionnels | A* (%) | B* (%) | C* (%) | D* (%) | E* (%) | F* (%) |
|---|---|---|---|---|---|---|---|---|
| Nature | Nombre d'équivalents | | | | | | | |
| $NaBO_3$ | 10 | Méthanol/eau/ NaOH | 100 | - | - | - | - | - |
| | 5 | Acide acétique | 2 | - | - | - | 19 | maj.* |
| $NaIO_4$/ $KMnO_4$ 1/1 | 1 | Acétone/eau | min.* | maj. | - | - | - | maj. |
| $KMnO_4$ (+catalyseur transfert de phase*) | 3 | Dichlorométhane | 100 | - | - | - | - | - |
| | 3 | Dichlorométhane/acide acétique | - | min. | - | - | min. | maj. |
| $H_2O_2$ | 6 | Méthanol/eau | maj. | traces | - | - | - | - |
| Oxone | 3 | Méthanol/eau | maj. | - | traces | - | maj. | min. |
| | 6 | Méthanol/eau | - | - | traces | - | maj. | min. |
| | 2 | Méthanol/eau/ $H_2SO_4$ | 9 | - | 2 | - | 48 | - |
| | 6 | Méthanol/eau/ $K_2CO_3$ | min. | 20 | 7 | - | - | - |

| | | | | | | | | |
|---|---|---|---|---|---|---|---|---|
| Acide chloro-3 perbenzoïque | 2 | Méthanol | 2 | 10 | 1 | 7 | 32 | min. |

2) Procédé de l'invention

| | | | | | | | | |
|---|---|---|---|---|---|---|---|---|
| Acide chloro-3 perbenzoïque | 4 | Méthanol/ $K_2CO_3$ | 29 | 9 | 53 | - | - | - |

* A : isopropyl-2 p-méthoxyphénylthio-3 indolizine

B : isopropyl-2 p-méthoxyphénylsulfoxide-3 indolizine

C : isopropyl-2 p-méthoxyphénylsulfonyl-3 indolizine

D : isopropyl-2 p-méthoxyphénylsulfoxide-1 indolizine

E : bis-(p-méthoxyphényl)-thiosulfonate

F : produits non identifiés

maj. : produit majoritaire

min. : produit minoritaire

catalyseur de transfert de phase : tris[(méthoxy-2 éthoxy)-2 éthyl]amine

Ces résultats montrent que seul le procédé de l'invention permet d'obtenir des rendements importants en dérivé sulfonyl-3 indolizine de formule I.

D'autre part, ces résultats mettent clairement en évidence l'importance d'un milieu basique pour une mise en oeuvre efficace du procédé d'oxydation de l'invention.

Comme indiqué précédemment, les dérivés de sulfonyl-3 indolizine de formule I peuvent être utilisés comme intermédiaires de synthèse pour la préparation des dérivés d'indolizine de formule Ia ci-dessus.

Ainsi les composés de formule Ia peuvent être obtenus par mise en oeuvre d'un procédé comprenant :

a) la déprotection du groupement p-hydroxyphénylsulfonyle

- lorsque $R_1$ représente méthyle par traitement au moyen d'un mélange éthanethiol/chlorure d'aluminium
- lorsque $R_1$ représente benzyle par traitement au moyen d'hydrogène en présence d'un catalyseur tel que le nickel de Raney ou le charbon palladié,
- lorsque $R_1$ représente un groupement alkylsulfonyle ou arylsulfonyle par traitement en milieu basique

pour obtenir les dérivés p-hydroxyphénylsulfonyles de formule générale :

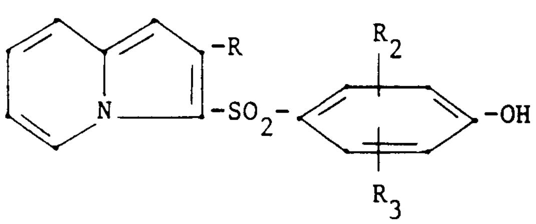

IV

dans laquelle R , $R_2$ et $R_3$ ont la même signification que précédemment,
b) la condensation du composé de formule IV avec un dihalogénoalkane de formule générale :

Hal-A-Hal V

dans laquelle A représente un radical alkylène tel que défini dans la formule Ia et Hal représente un atome d'halogène et ce, au reflux, dans un solvant tel que la méthyl-éthyl-cétone ou le N,N-diméthylformamide et en présence d'un agent basique,
ou bien
$b_1$) la condensation avec un alcool halogéné de formule générale :

Hal-A-OH VI

dans laquelle A représente un radical alkylène tel que défini dans la formule Ia et Hal a la même signification que précédemment, et ce, dans un solvant tel que le N,N-diméthylformamide et en présence d'un agent basique puis condensation du dérivé alcool obtenu avec un halogénure de formule générale :

Hal-W VII

dans laquelle W représente un radical alkylsulfonyle en $C_1$-$C_4$ par exemple méthanesulfonyle ou arylsulfonyle en $C_6$-$C_{10}$ par exemple benzènesulfonyle ou p-toluènesulfonyle, dans un solvant accepteur d'acide par exemple la pyridine
ou bien
$b_2$) le chauffage au reflux avec une épihalohydrine telle que l'épichlorhydrine ou l'épibromhydrine en présence d'un agent basique et dans un solvant polaire tel que la méthyl-éthyl-cétone
pour obtenir les dérivés de sulfonyl-3 indolizine de formule générale :

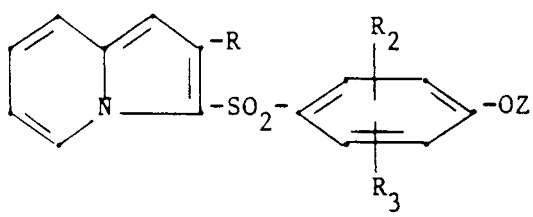

VIII

dans laquelle R, $R_2$ et $R_3$ ont la même signification que précédemment et Z représente un radical oxyranylméthyle ou un radical de formule :

$-A-Z_1$

dans laquelle A représente un radical alkylène, linéaire ou ramifié, en $C_2$-$C_5$ et $Z_1$ représente un atome d'halogène, un radical alkylsulfonyloxy en $C_1$-$C_4$ ou arylsulfonyloxy en $C_6$-$C_{10}$.

L'agent basique utilisé lors du traitement du composé de formule IV est généralement un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium.
c) On fait alors réagir le dérivé de formule VIII avec une amine de formule générale :

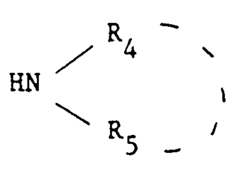

IX

dans laquelle $R_4$ et $R_5$ ont la même signification que précédemment, la réaction ayant lieu en présence

d'un accepteur d'acide et dans un solvant approprié généralement un solvant polaire tel qu'un alcool par exemple le butanol, une cétone telle que la méthyl-éthyl-cétone, un hydrocarbure aromatique par exemple le benzène, le toluène ou un xylène ou encore un excès d'amine de formule IX pour obtenir les composés de formule Ia sous forme de base libre, que l'on peut faire réagir, si on le désire, avec un acide approprié pour former un sel pharmaceutiquement acceptable de ce composé.

Suivant une méthode alternative, on peut également mettre en oeuvre les composés de formule IV en traitant directement un tel composé avec un halogénure de formule générale :

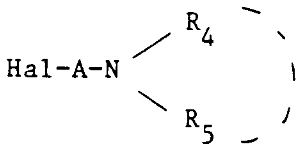

X

dans laquelle Hal, $R_4$ et $R_5$ ont la même signification que précédemment et A représente un radical alkylène en $C_2$-$C_5$ tel que défini précédemment, la réaction étant conduite en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium pour obtenir les composés de formule Ia dans laquelle A représente un radical alkylène en $C_2$-$C_5$.

Les Exemples suivants illustrent le procédé de l'invention ainsi que l'utilisation des dérivés d'indolizine de formule I pour la préparation des composés de formule Ia :

## EXEMPLE 1

### Préparation de l'isopropyl-2 p-méthoxyphénylsulfonyl-3 indolizine.

a) Isopropyl-2 p-méthoxyphénylthio-3 indolizine

On chauffe, à reflux durant 90h un mélange de 25,2g (0,158 mole) d'isopropyl-2 indolizine et de 44g (0,158 mole) de bis-(méthoxy-4 phényl) disulfure dans 500ml d'éthanol.

Après réaction, on laisse refroidir et on évapore à sec. On reprend le résidu dans l'éther et on extrait avec une solution d'hydroxyde de sodium à 5%.
On lave à l'eau, on sèche et on décolore avec du charbon actif. On filtre, on évapore à sec et on purifie, le résidu obtenu, par chromatographie d'élution sur silice en utilisant le tétrachlorure de carbone comme éluant.

De cette manière, on obtient 14g d'isopropyl-2 p-méthoxyphénylthio-3 indolizine sous forme de cristaux après recristallisation dans l'hexane.

Rendement : 27%
Pureté : 98,3% (chromatographie liquide haute pression).
P.F. : 59-60 °C.

b) Isopropyl-2 p-méthoxyphénylsulfonyl-3 indolizine

On dissout 1g ($3,36.10^{-3}$ mole) d'isopropyl-2 p-méthoxyphénylthio-3 indolizine dans 60ml de méthanol puis on ajoute d'abord 3,36g ($33,6.10^{-3}$ mole) de carbonate de potassium, ensuite 2,4g ($12,34.10^{-3}$ mole) d'acide chloro-3 perbenzoïque à 88,7%. On agite durant 4 heures à température ambiante, on verse le mélange dans 100ml d'eau puis on extrait plusieurs fois avec de l'acétate d'éthyle. On sèche la phase organique sur sulfate de magnésium anhydre, on filtre et on concentre sous vide. On élue l'huile obtenue sur colonne de silice en utilisant un mélange acétate d'éthyle/hexane 30/70 comme éluant.

De cette manière, on obtient l'isopropyl-2 p-méthoxyphénylsulfonyl-3 indolizine avec un rendement de 53%.

P.F. : 103,5 °C (isopropanol)

| Analyse centésimale | | | | |
|---|---|---|---|---|
| | C% | H% | N% | S% |
| Calculé<br>Trouvé | 65,63<br>65,44 | 5,81<br>5,76 | 4,25<br>4,33 | 9,73<br>9,86 |

Spectre I.R. (KBr) :

1320 $cm^{-1}$ : stretching asymétrique de $SO_2$
1145 $cm^{-1}$ : stretching symétrique de $SO_2$

Spectre R.M.N. :

$\delta$ : 1,1-1,5 ppm (6H).
3,6-4,2 ppm (4H)
6,3-6,5 ppm (1H)
6,5-7,1 ppm (4H)
7,2-7,5 ppm (1H)
7,6-7,9 ppm (2H)
8,7-8,9 ppm (1H)

L'Exemple suivant illustre la préparation d'un dérivé d'aminoalkoxyphénylsulfonyl-3 indolizine de formule Ia :

EXEMPLE I

Préparation de l'oxalate d'isopropyl-2 [{[ N-méthyl-N-(diméthoxy-3,4 $\beta$-phénéthyl)amino]-3 propyloxy}-4 phénylsulfonyl]-3 indolizine.

a) Isopropyl-2 p-hydroxyphénylsulfonyl-3 indolizine

Dans 100ml de dichlorométhane et 25ml d'éthanethiol, on met en suspension 6,7g (0,050 mole) de chlorure d'aluminium. On agite cette suspension et on la refroidit à 0°C tout en ajoutant 2,5g d'isopropyl-2 p-méthoxyphénylsulfonyl-3 indolizine dans du dichlorométhane. L'addition prend environ 15 minutes. On ramène alors le milieu réactionnel à température ambiante et on l'y maintient pendant 45 minutes. On le coule sur de la glace puis on y ajoute sous agitation 5ml d'acide chlorhydrique concentré. On extrait avec 2 fractions d'éther éthylique et on réunit les extraits éthérés. On lave avec 3 fractions de 30ml d'une solution aqueuse de carbonate de sodium à 10% et on acidifie la phase aqueuse.

On obtient de cette manière l'isopropyl-2 p-hydroxyphénylsulfonyl-3 indolizine sous forme brute.

b) Oxalate d'isopropyl-2 [{[ N-méthyl-N-(diméthoxy-3,4 $\beta$-phénéthyl)amino]-3 propyloxy}-4 phénylsulfonyl]-3 indolizine.

On agite, à température ambiante durant 30 minutes, 0,510g (1,57. $10^{-3}$ mole) d'isopropyl-2 p-hydroxyphénylsulfonyl-3 indolizine, 0,5g de carbonate de potassium et 5ml de diméthylsulfoxyde. A ce mélange, on ajoute alors 0,524g (1,45.$10^{-3}$ mole) d'oxalate acide de chloro-1 [N-méthyl-N-(diméthoxy-3,4 $\beta$-phénéthyl)amino]-3 propane. On maintient l'agitation durant 26 h à température ambiante puis durant 2 h à 50°C. On élimine sous vide le diméthylsulfoxyde et on reprend le résidu dans l'eau. On extrait deux fois le milieu avec de l'acétate d'éthyle, on lave deux fois les extraits avec de l'eau et on sèche sur sulfate de sodium.

Après filtration, on évapore le filtrat sous vide pour obtenir 0,845g d'une huile couleur ambre. On purifie cette huile sur colonne de silice en utilisant, comme éluants, de l'acétate d'éthyle contenant 5%, puis 10% puis 20% de méthanol, ce qui fournit 0,583g du produit désiré sous forme de base libre.

(rendement : 73% ; pureté : 99,4%).

On forme alors l'oxalate en utilisant 0,530g de la base ainsi obtenue et une solution d'acide oxalique dans l'éther éthylique. On recristallise ensuite l'oxalate formé dans un mélange acétate d'éthyle/méthanol/éther éthylique.

De cette manière, on obtient 0,473g d'oxalate d'isopropyl-2 [{[N-méthyl-N-(diméthoxy-3,4 $\beta$-phénéthyl)-amino]-3 propyloxy}-4 phénylsulfonyl]-3 indolizine sous forme d'un solide blanc.

P.F. : 135-137°C.

**Revendications**

**Revendications pour les Etats contractants suivants : AT, BE, CH, DE, FR, GB, GR, IT, LI, LU, NL, SE**

1. Dérivés de sulfonyl-3 indolizine de formule générale :

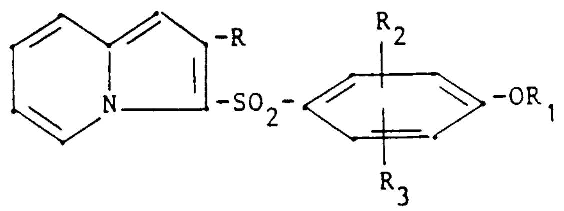

I

dans laquelle :
R représente l'hydrogène, un radical alkyle, linéaire ou ramifié en $C_1$-$C_8$, un radical cycloalkyle en $C_3$-$C_6$ ou un groupement phényle non substitué ou substitué par un ou plusieurs substituants, identiques ou différents, choisis parmi des atomes d'halogène et parmi des groupements alkyle en $C_1$-$C_4$, alkoxy en $C_1$-$C_4$ ou nitro,
$R_1$ repésente un radical protecteur d'un groupement hydroxyle, à savoir un groupement méthyle, benzyle, alkylsulfonyle en $C_1$-$C_4$ ou arylsulfonyle en $C_6$-$C_{10}$,
$R_2$ et $R_3$, identiques ou différents, représentent chacun l'hydrogène, le radical méthyle ou éthyle ou un atome d'halogène.

2. Dérivés de sulfonyl-3 indolizine selon la revendication 1 dans laquelle R représente un groupe isopropyle ou cyclopropyle.

3. Dérivés de sulfonyl-3 indolizine selon la revendication 1 ou 2 dans laquelle $R_1$ représente un groupe méthyle.

4. Isopropyl-2 p-méthoxyphénylsulfonyl-3 indolizine.

5. Procédé de préparation de dérivés de sulfonyl-3 indolizine selon l'une quelconque des revendications 1 à 4, caractérisé en ce que l'on oxyde à température ambiante un dérivé phénylthio-3 indolizine de formule générale :

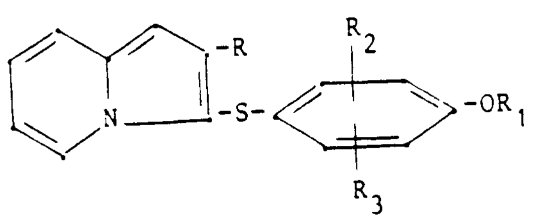

II

dans laquelle R, $R_1$, $R_2$ et $R_3$ ont la même signification que précedemment, au moyen d'acide chloro-3 perbenzoïque, en présence d'un agent basique et dans un alcool inférieur en $C_1$-$C_4$ comme solvant.

6. Procédé selon la revendication 5, caractérisé en ce que l'agent basique est un carbonate de métal alcalin.

7. Procédé selon la revendication 5, caractérisé en ce que le solvant est le méthanol.

8. Procédé selon l'une quelconque des revendications 5 à 7, caractérisé en ce que l'on utilise de 1 à 5 équivalents d'acide chloro-3 perbenzoïque par équivalent de dérivé phénylthio-3 indolizine de formule II.

**9.** Procédé selon la revendication 8, caractérisé en ce que l'on utilise 4 équivalents d'acide chloro-3 perbenzoïque.

**10.** Procédé selon une des revendications 5 à 9, caractérisé en ce que l'on utilise de 2 à 4 équivalents d'agent basique par équivalent d'acide chloro-3 perbenzoïque.

**11.** Procédé de préparation d'un dérivé d'aminoalkoxyphénylsulfonyl-3 indolizine de formule générale :

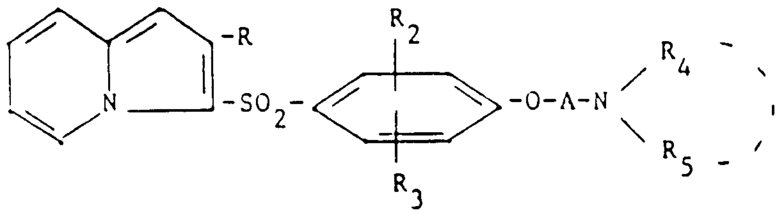

Ia

ainsi que ses sels pharmaceutiquement acceptables, dans laquelle R, $R_2$ et $R_3$ ont la même signification que dans la revendication 1,
A représente un radical alkylène, linéaire ou ramifié en $C_2$-$C_5$ ou le radical hydroxy-2 propylène dans lequel l'hydroxy est éventuellement substitué par un radical alkyle en $C_1$-$C_4$.
$R_4$ représente un radical alkyle linéaire ou ramifié en $C_1$-$C_8$ ou un radical de formule :

-Alk-$R_6$

dans laquelle Alk représente un liaison simple ou un radical alkylène, linéaire ou ramifié, en $C_1$-$C_5$ et $R_6$ représente un radical pyridyle, phényle, méthylènedioxy-2,3-phényle, méthylènedioxy-3,4-phényle, ou un groupement phényle, substitué par un ou plusieurs substituants identiques ou différents choisis parmi des atomes d'halogène, des groupements alkyle en $C_1$-$C_4$ ou des groupements alkoxy en $C_1$-$C_4$, $R_5$ représente l'hydrogène ou un radical alkyle en $C_1$-$C_8$ ou $R_4$ et $R_5$, lorsqu'ils sont pris ensemble représentent un radical alkylène ou alkénylène en $C_3$-$C_6$ éventuellement substitué par un radical phényle ou
éventuellement interrompu par -O-, -N= ou -N-$R_7$, $R_7$ représentant l'hydrogène, un radical alkyle en $C_1$-$C_4$ ou un radical phényle, caractérisé en ce que

a) on effectue une déprotection du groupement p-hydroxyphénylsulfonyle d'un composé de formule I tel que défini à la revendication 1 pour obtenir un dérivé p-hydroxyphénylsulfonyle de formule générale :

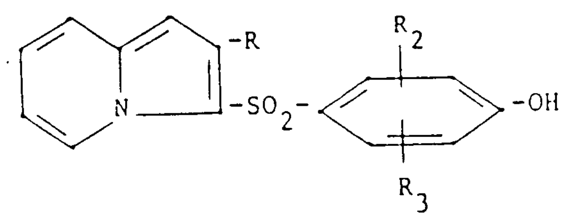

IV

dans laquelle R, $R_2$ et $R_3$ ont la même signification que précédemment,

b) on effectue la condensation du composé de formule IV avec un dihalogénoalkane de formule générale :

Hal-A-Hal V

dans laquelle A représente un radical alkylène tel que défini dans la formule Ia et Hal représente un atome d'halogène et ce, au reflux, dans un solvant tel que la méthyl-éthyl-cétone ou le N,N-diméthylformamide et en présence d'un agent basique,
<u>ou bien</u>

$b_1$) la condensation avec un alcool halogéné de formule générale :

Hal-A-OH VI

dans laquelle A représente un radical alkylène tel que défini dans la formule Ia et Hal a la même signification que précédemment, et ce, dans un solvant tel que le N,N-diméthylformamide et en présence d'un agent basique puis la condensation du dérivé alcool obtenu avec un halogénure de formule générale :

Hal-W VII

dans laquelle W représente un radical alkylsulfonyle en $C_1$-$C_4$ ou arylsulfonyle en $C_6$-$C_{10}$, dans un solvant accepteur d'acide
ou bien
$b_2$) le chauffage au reflux avec une épihalohydrine telle que l'épichlorhydrine ou l'épibromhydrine en présence d'un agent basique et dans un solvant polaire,
pour obtenir un dérivé de sulfonyl-3 indolizine de formule générale :

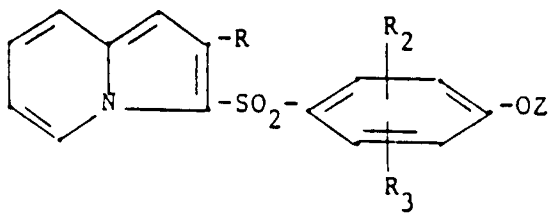

VIII

dans laquelle R, $R_2$ et $R_3$ ont la même signification que précédemment et Z représente un radical oxyranylméthyle ou un radical de formule :

-A-$Z_1$

dans laquelle A représente un radical alkylène, linéaire ou ramifié, en $C_2$-$C_5$ et $Z_1$ représente un atome d'halogène, un radical alkylsulfonyloxy en $C_1$-$C_4$ ou arylsulfonyloxy en$C_6$-$C_{10}$.

c) on fait réagir le dérivé de formule VIII avec une amine de formule générale

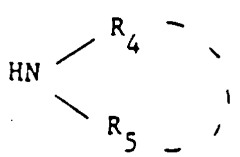

IX

dans laquelle $R_4$ et $R_5$ ont la même signification que précédemment, la réaction ayant lieu en présence d'un accepteur d'acide et dans un solvant approprié, pour obtenir un composé de formule Ia sous forme de base libre, que l'on peut faire réagir, si on le désire, avec un acide approprié pour former un sel pharmaceutiquement acceptable de ce composé.

**12.** Procédé de préparation d'un dérivé d'aminoalkoxyphénylsulfonyl-3 indolizine de formule générale :

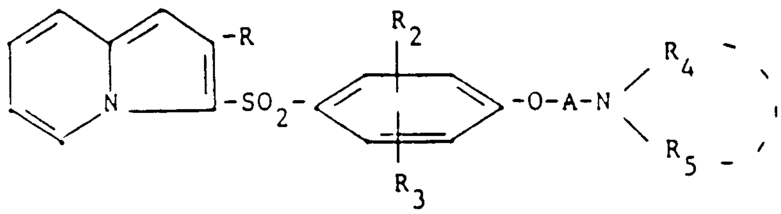

Ia

ainsi que de ses sels pharmaceutiquement acceptables, dans laquelle R, $R_2$ et $R_3$ ont la même signification qu'à la revendication 1,
A représente un radical alkylène, linéaire ou ramifié en $C_2$-$C_5$
$R_4$ représente un radical alkyle linéaire ou ramifié en $C_1$-$C_8$ ou un radical de formule :

-Alk-$R_6$

dans laquelle
Alk représente un liaison simple ou un radical alkylène, linéaire ou ramifié, en $C_1$-$C_5$ et $R_6$ représente un radical pyridyle, phényle, méthylènedioxy-2,3 phényle, méthylènedioxy-3,4 phényle ou un groupement phényle substitué par un ou plusieurs substituants identiques ou différents choisis parmi des atomes d'halogène, des groupements alkyle en $C_1$-$C_4$ ou des groupements alkoxy en $C_1$-$C_4$,

$R_5$ représente l'hydrogène ou un radical alkyle en $C_1$-$C_8$ ou $R_4$ et $R_5$, lorsqu'ils sont pris ensemble représentent un radical alkylène ou alkénylène en $C_3$-$C_6$ éventuellement substitué par un radical phényle ou éventuellement interrompu par -O-, -N= ou -N-$R_7$, $R_7$ représentant l'hydrogène, un radical alkyle en $C_1$-$C_4$ ou un radical phényle,
caractérisé en ce que

a) l'on effectue une déprotection du groupement p-hydroxyphénylsulfonyle d'un composé de formule I tel que défini à la revendication 1 pour obtenir un dérivé p-hydroxyphénylsulfonyle de formule générale :

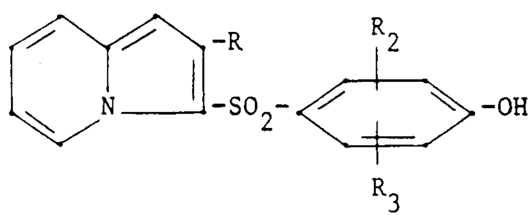

IV

dans laquelle R, $R_2$ et $R_3$ ont la même signification que précédemment, et

b) l'on traite le composé de formule IV avec un halogénure de formule générale :

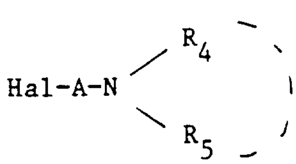

X

dans laquelle Hal, $R_4$ et $R_5$ ont la même signification que précédemment et A représente un radical alkylène en $C_2$-$C_5$,la réaction étant conduite en présence d'un agent basique.

**Revendications pour l'Etat contractant suivant : ES**

**1.** Procédé de préparation de dérivés de sulfonyl-3 indolizine de formule générale :

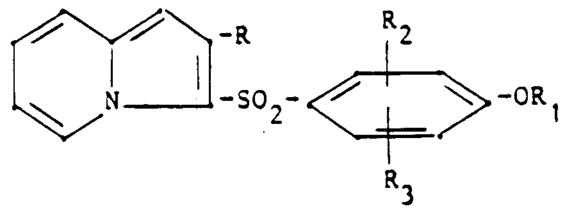

I

dans laquelle :
R représente l'hydrogène, un radical alkyle, linéaire ou ramifié en $C_1$-$C_8$, un radical cycloalkyle en $C_3$-$C_6$ ou un groupement phényle non substitué ou substitué par un ou plusieurs substituants, identiques ou différents, choisis parmi des atomes d'halogène et parmi des groupements alkyle en $C_1$-$C_4$, alkoxy en $C_1$-$C_4$ ou nitro,
$R_1$ représente un radical protecteur d'un groupement hydroxyle, à savoir un groupement méthyle, benzyle, alkylsulfonyle en $C_1$-$C_4$ ou arylsulfonyle en $C_6$-$C_{10}$; $R_2$ et $R_3$, identiques ou différents, représentent chacun l'hydrogène, le radical méthyle ou éthyle ou un atome d'halogène; caractérisé en ce que l'on oxyde à température ambiante un dérivé phénylthio-3 indolizine de formule générale :

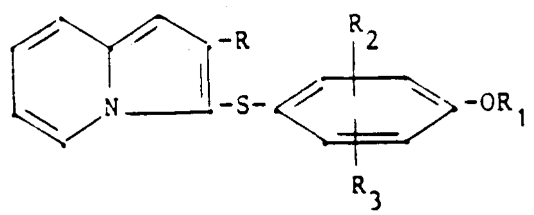

II

dans laquelle R, $R_1$, $R_2$ et $R_3$ ont la même signification que précédemment, au moyen d'acide chloro-3 perbenzoïque, en présence d'un agent basique et dans un alcool inférieur en $C_1$-$C_4$ comme solvant.

**2.** Procédé selon la revendication 1, caractérisé en ce que l'agent basique est un carbonate de métal alcalin.

**3.** Procédé selon la revendication 1, caractérisé en ce que le solvant est le méthanol.

**4.** Procédé selon l'une quelconque des revendications 1 à 3, caractérisé en ce que l'on utilise de 1 à 5 équivalents d'acide chloro-3 perbenzoïque par équivalent de dérivé phénylthio-3 indolizine de formule II.

**5.** Procédé selon la revendication 4, caractérisé en ce que l'on utilise 4 équivalents d'acide chloro-3 perbenzoïque.

**6.** Procédé selon une des revendications 1 à 5, caractérisé en ce que l'on utilise de 2 à 4 équivalents d'agent basique par équivalent d'acide chloro-3 perbenzoïque.

**7.** Procédé de préparation d'un dérivé d'aminoalkoxyphénylsulfonyl-3 indolizine de formule générale :

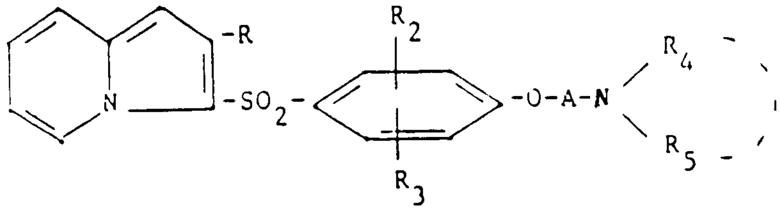

I a

ainsi que ses sels pharmaceutiquement acceptables, dans laquelle R, $R_2$ et $R_3$ ont la même signification que dans la revendication 1,
A représente un radical alkylène, linéaire ou ramifié en $C_2$-$C_5$ ou le radical hydroxy-2 propylène dans lequel l'hydroxy est éventuellement substitué par un radical alkyle en $C_1$-$C_4$,
$R_4$ représente un radical alkyle linéaire ou ramifié en $C_1$-$C_8$ ou un radical de formule :

-Alk-$R_6$

dans laquelle Alk représente un liaison simple ou un radical alkylène, linéaire ou ramifié, en $C_1$-$C_5$ et $R_6$ représente un radical pyridyle, phényle, méthylènedioxy-2,3-phényle, méthylènedioxy-3,4-phényle, ou un groupement phényle, substitué par un ou plusieurs substituants identiques ou différents choisis parmi des atomes d'halogène, des groupements alkyle en $C_1$-$C_4$ ou des groupements alkoxy en $C_1$-$C_4$,
$R_5$ représente l'hydrogène ou un radical alkyle en $C_1$-$C_8$ ou $R_4$ et $R_5$, lorsqu'ils sont pris ensemble représentent un radical alkylène ou alkénylène en $C_3$-$C_6$ éventuellement substitué par un radical phényle ou
éventuellement interrompu par -O-,-N= ou $-\overset{|}{N}-R_7$, $R_7$ représentant l'hydrogène, un radical alkyle en $C_1$-$C_4$ ou un radical phényle, caractérisé en ce que

a) on effectue une déprotection du groupement p-hydroxyphénylsulfonyle d'un composé de formule I tel que défini à la revendication 1 pour obtenir un dérivé p-hydroxyphénylsulfonyle de formule générale :

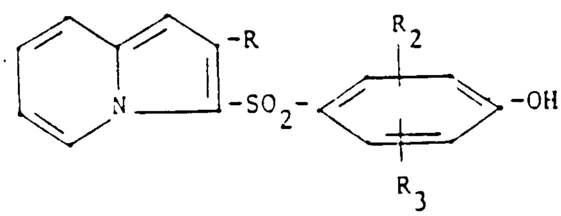

IV

dans laquelle R, $R_2$ et $R_3$ ont la même signification que précédemment,

b) on effectue la condensation du composé de formule IV avec un dihalogénoalkane de formule générale :

Hal-A-Hal V

dans laquelle A représente un radical alkylène tel que défini dans la formule Ia et Hal représente un atome d'halogène et ce, au reflux, dans un solvant tel que la méthyl-éthyl-cétone ou le N,N-diméthylformamide et en présence d'un agent basique,

<u>ou bien</u>

$b_1$) la condensation avec un alcool halogéné de formule générale :

Hal-A-OH VI

dans laquelle A représente un radical alkylène tel que défini dans la formule Ia et Hal a la même

signification que précédemment, et ce, dans un solvant tel que le N,N-diméthylformamide et en présence d'un agent basique puis la condensation du dérivé alcool obtenu avec un halogénure de formule générale :

Hal-W VII

dans laquelle W représente un radical alkylsulfonyle en $C_1$-$C_4$ ou arylsulfonyle en $C_6$-$C_{10}$, dans un solvant accepteur d'acide

ou bien

$b_2$) le chauffage au reflux avec une épihalohydrine telle que l'épichlorhydrine ou l'épibromhydrine en présence d'un agent basique et dans un solvant polaire,

pour obtenir un dérivé de sulfonyl-3 indolizine de formule générale :

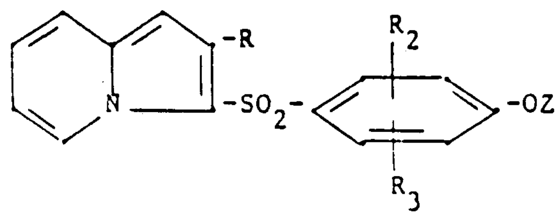

VIII

dans laquelle R, $R_2$ et $R_3$ ont la même signification que précédemment et Z représente un radical oxyranylméthyle ou un radical de formule :

$-A-Z_1$

dans laquelle A représente un radical alkylène, linéaire ou ramifié, en $C_2$-$C_5$ et $Z_1$ représente un atome d'halogène, un radical alkylsulfonyloxy en $C_1$-$C_4$ ou arylsulfonyloxy en$C_6$-$C_{10}$.

c) on fait réagir le dérivé de formule VIII avec une amine de formule générale

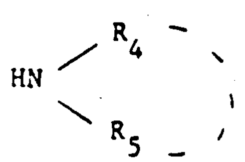

IX

dans laquelle $R_4$ et $R_5$ ont la même signification que précédemment, la réaction ayant lieu en présence d'un accepteur d'acide et dans un solvant approprié, pour obtenir un composé de formule la sous forme de base libre, que l'on peut faire réagir, si on le désire, avec un acide approprié pour former un sel pharmaceutiquement acceptable de ce composé.

**8.** Procédé de préparation d'un dérivé d'aminoalkoxyphénylsulfonyl-3 indolizine de formule générale :

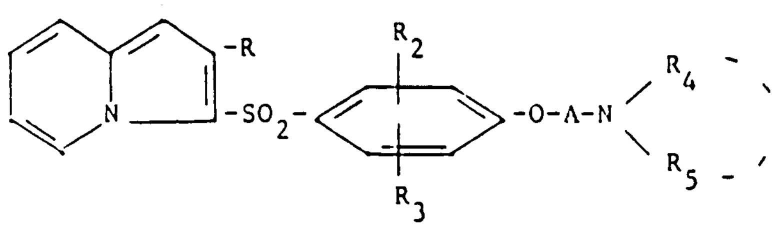

Ia

ainsi que de ses sels pharmaceutiquement acceptables, dans laquelle R, $R_2$ et $R_3$ ont la même signification qu'à la revendication 1,

A représente un radical alkylène, linéaire ou ramifié en $C_2$-$C_5$

$R_4$ représente un radical alkyle linéaire ou ramifié en $C_1$-$C_8$ ou un radical de formule :

-Alk-$R_6$

dans laquelle
Alk représente un liaison simple ou un radical alkylène, linéaire ou ramifié, en $C_1$-$C_5$ et $R_6$ représente un radical pyridyle, phényle, méthylènedioxy-2,3 phényle, méthylènedioxy-3,4 phényle ou un groupement phényle substitué par un ou plusieurs substituants identiques ou différents choisis parmi des atomes d'halogène, des groupements alkyle en $C_1$-$C_4$ ou des groupements alkoxy en $C_1$-$C_4$,

$R_5$ représente l'hydrogène ou un radical alkyle en $C_1$-$C_8$ ou $R_4$ et $R_5$, lorsqu'ils sont pris ensemble représentent un radical alkylène ou alkénylène en $C_3$-$C_6$ éventuellement substitué par un radical phényle ou éventuellement interrompu par -O-, -N= ou -N-$R_7$, $R_7$ représentant l'hydrogène, un radical alkyle en $C_1$-$C_4$ ou un radical phényle,
caractérisé en ce que

a) l'on effectue une déprotection du groupement p-hydroxyphénylsulfonyle d'un composé de formule I tel que défini à la revendication 1 pour obtenir un dérivé p-hydroxyphénylsulfonyle de formule générale :

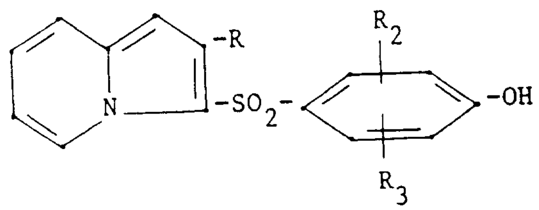

IV

dans laquelle R, $R_2$ et $R_3$ ont la même signification que précédemment, et

b) l'on traite le composé de formule IV avec un halogénure de formule générale :

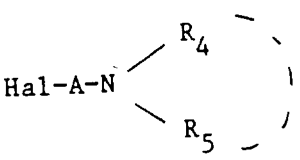

X

dans laquelle Hal, $R_4$ et $R_5$ ont la même signification que précédemment et A représente un radical alkylène en $C_2$-$C_5$,la réaction étant conduite en présence d'un agent basique.

**9.** Procédé de préparation de dérivés de sulfonyl -3 indolizine selon la revendication 1, caractérisé en ce que l'on prépare un dérivé de formule I dans laquelle R représente un groupe isopropyle ou cyclopropyle.

**10.** Procédé de préparation de dérivés de sulfonyl-3 indolizine selon la revendication 1 ou la revendication 9, caractérisé en ce que l'on prépare un dérivé de formule I dans laquelle $R_1$ représente un groupe méthyle.

**11.** Procédé de préparation de dérivés de sulfonyl-3 indolizine selon la revendication 9, caractérisé en ce que l'on prépare l'isopropyl-2 p-méthoxyphénylsulfonyl-3 indolizine.

**Claims**

**Claims for the following Contracting States : AT, BE, CH, DE, FR, GB, GR, IT, LI, LU, NL, SE**

1. 3-Sulphonyl indolizine derivatives of general formula:

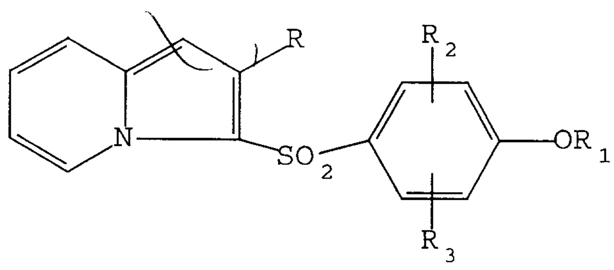

I

in which:
R represents hydrogen, a linear or branched $C_1$-$C_8$ alkyl radical, a $C_3$-$C_6$ cycloalkyl radical, an unsubstituted phenyl group or a phenyl group substituted by one or more substituents, identical or different, selected from halogen atoms, $C_1$-$C_4$ alkyl groups, $C_1$-$C_4$ alkoxy groups or the nitro group,
$R_1$ represents a protecting group for hydroxyl, namely a methyl, benzyl, $C_1$-$C_4$ alkylsulphonyl or $C_6$-$C_{10}$ arylsulphonyl group,
$R_2$ and $R_3$, which may be identical or different, each represents hydrogen, a methyl or ethyl group, or a halogen atom.

2. 3-Sulphonyl indolizine derivatives according to claim 1 in which R represents an isopropyl or cyclopropyl group.

3. 3-Sulphonyl indolizine derivatives according to claim 1 or 2 in which $R_1$ represents a methyl group.

4. 2-Isopropyl-3-p-methoxyphenylsulphonyl indolizine.

5. A process for the preparation of 3-sulphonyl indolizine derivatives according to any one of claims 1 to 4, characterised in that a 3-phenylthio indolizine derivative of general formula:

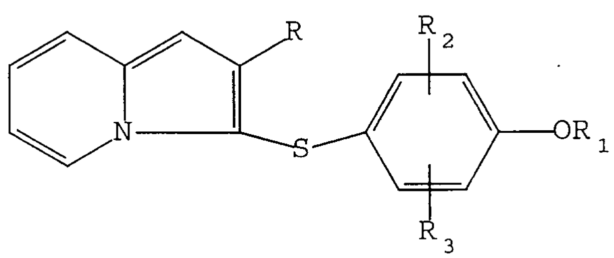

II

in which R, $R_1$, $R_2$ and $R_3$ are as defined previously, is oxidised at room temperature by means of 3-chloroperbenzoic acid in the presence of a basic reagent and in a $C_1$-$C_4$ lower alcohol as solvent.

6. A process according to claim 5, characterised in that the basic reagent is an alkali metal carbonate.

7. A process according to claim 5, characterised in that the solvent is methanol.

8. A process according to any of claims 5 to 7, characterised in that 1 to 5 equivalents of 3-chloroperbenzoic acid are used per equivalent of 3-phenylthio indolizine derivative of formula II.

9. A process according to claim 8, characterised in that 4 equivalents of 3-chloroperbenzoic acid are used.

10. A process according to one of claims 5 to 9, characterised in that 2 to 4 equivalents of basic reagent are used per equivalent of 3-chloroperbenzoic acid.

**11.** A process for the preparation of a 3-aminoalkoxyphenyl-sulphonyl indolizine derivative of general formula:

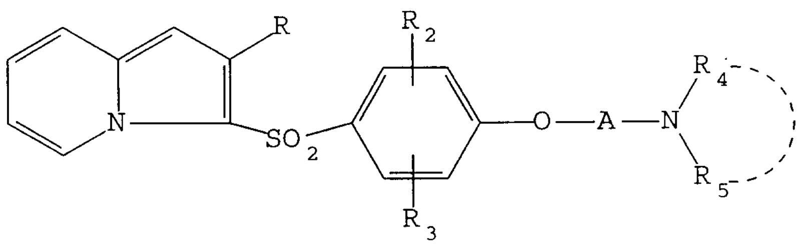

Ia

as well as its pharmaceutically acceptable salts, in which R, $R_2$ and $R_3$ are as defined in claim 1,
A represents a linear or branched $C_2$-$C_5$ alkylene radical, or a 2-hydroxy propylene radical in which the hydroxy is optionally substituted by a $C_1$-$C_4$ alkyl radical,
$R_4$ represents a linear or branched $C_1$-$C_8$ alkyl radical or a radical of formula:

- Alk-$R_6$

in which Alk represents a single bond or a linear or branched $C_1$-$C_5$ alkylene radical, and $R_6$ represents a pyridyl, phenyl, 2,3-methylenedioxyphenyl, or 3,4-methylenedioxyphenyl radical or a phenyl group substituted by one or more substituents, identical or different, selected from halogen atoms, $C_1$-$C_4$ alkyl groups or $C_1$-$C_4$ alkoxy groups,
$R_5$ represents hydrogen or a $C_1$-$C_8$ alkyl radical, or $R_4$ and $R_5$, when they are taken together, represent a $C_3$-$C_6$ alkylene or alkenylene radical, optionally substituted by a phenyl radical or optionally interrupted by -O-, -N= or -N-$R_7$,
$R_7$ representing hydrogen, a $C_1$-$C_4$ alkyl group or a phenyl group, characterised in that

a) deprotection of the p-hydroxyphenylsulphonyl group of the compound of formula I as defined previously is carried out in order to obtain a p-hydroxyphenylsulphonyl derivative of general formula:

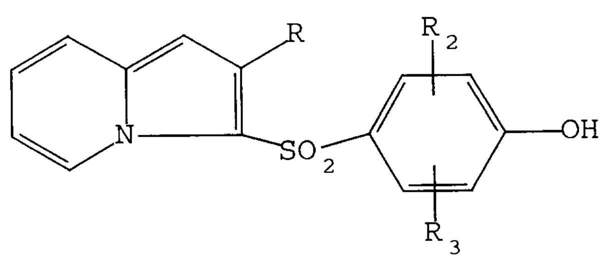

IV

in which R, $R_2$ and $R_3$ are as defined previously;
b) the condensation of the compound of formula IV is carried out with an alkane dihalide of general formula:

Hal-A-Hal V

in which A represents an alkylene radical as defined in formula Ia and Hal represents a halogen atom, under reflux in a solvent such as methyl ethyl ketone or N,N-dimethylformamide and in the presence of a basic reagent,
<u>or alternatively</u>

b₁) condensation with a halogenated alcohol of general formula:

Hal-A-OH VI

in which A represents an alkylene radical as defined in formula Ia and Hal is as defined previously, is carried out in a solvent such as N,N-dimethylformamide and in the presence of a basic reagent, followed by the condensation of the alcohol derivative obtained with a halide of

general formula:

Hal-W VII

in which W represents a $C_1$-$C_4$ alkylsulphonyl or $C_6$-$C_{10}$ arylsulphonyl radical, in an acid-acceptor solvent,
<u>or alternatively</u>
$b_2$) heating under reflux with an epihalohydrin such as epichlorhydrin or epibromohydrin in the presence of a basic reagent and in a polar solvent
in order to obtain a 3-sulphonyl indolizine derivative of general formula:

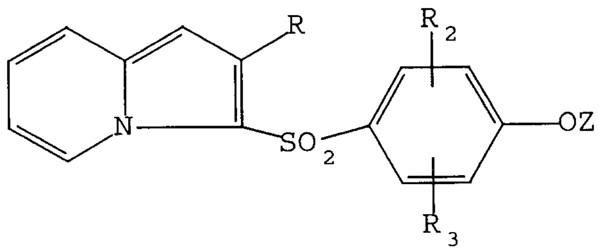

VIII

in which R, $R_2$ and $R_3$ are as defined previously and Z represents an oxiranylmethyl radical or a radical of formula:

$-A-Z_1$

in which A represents a linear or branched $C_2$-$C_5$ alkyleneradical, and $Z_1$ represents a halogen atom, a $C_1$-$C_4$ alkylsulphonyloxy radical, or a $C_6$-$C_{10}$ arylsulphonyloxy radical.

c) the derivative of formula VIII is reacted with an amine of general formula:

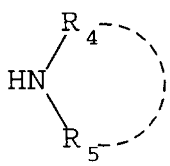

IX

in which $R_4$ and $R_5$ are as defined previously, the reaction taking place in the presence of an acid-acceptor in a suitable solvent in order to obtain a compound of formula Ia in the form of the free base, which may be reacted, if desired, with a suitable acid in order to form a pharmaceutically acceptable salt of this compound.

**12.** A process for the preparation of a 3-aminoalkoxyphenyl-sulphonyl indolizine derivative of general formula:

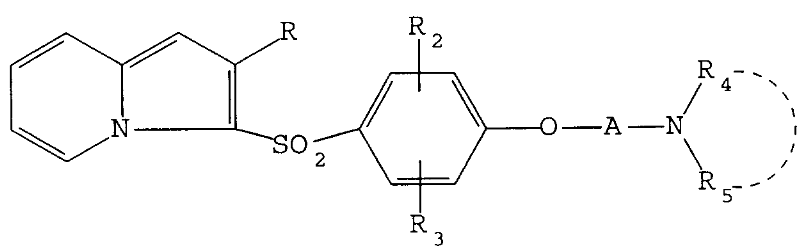

Ia

as well as its pharmaceutically acceptable salts, in which R, $R_2$ and $R_3$ are as defined in claim 1,
A represents a linear or branched $C_2$-$C_5$ alkylene radical,
$R_4$ represents a linear or branched $C_1$-$C_8$ alkyl radical or a radical of formula:

$-Alk-R_6$

in which

Alk represents a single bond or a linear or branched $C_1$-$C_5$ alkylene radical, and $R_6$ represents a pyridyl, phenyl, 2,3-methylenedioxyphenyl, or 3,4-methylenedioxyphenyl radical, or a phenyl group substituted by one or more substituents, identical or different, selected from halogen atoms, $C_1$-$C_4$ alkyl groups or $C_1$-$C_4$ alkoxy groups, $R_5$ represents hydrogen or a $C_1$-$C_8$ alkyl radical or $R_4$ and $R_5$, when they are taken together, represent a $C_3$-$C_6$ alkylene or alkenylene radical optionally substituted by a phenyl radical or optionally interrupted by -O-, $-N=$ or $-\overset{|}{N}-R_7$,

$R_7$ representing hydrogen, a $C_1$-$C_4$ alkyl radical or a phenyl radical, characterised in that

a) deprotection of the p-hydroxyphenylsulphonyl group of a compound of formula I as defined in claim 1 is carried out in order to obtain a p-hydroxyphenylsulphonyl derivative of general formula:

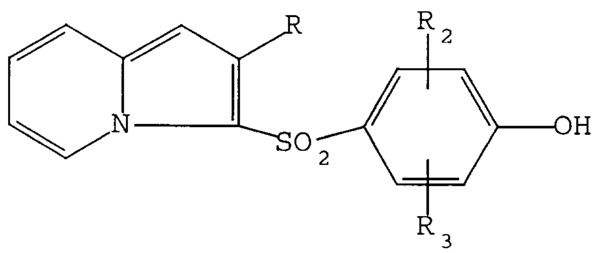

IV

in which R, $R_2$ and $R_3$ are as defined previously, and

b) the compound of formula IV is treated with a halide of general formula:

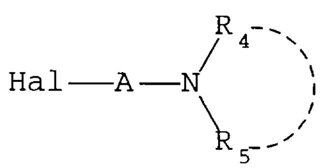

X

in which Hal, $R_4$ and $R_5$ are as defined previously and A represents a $C_2$-$C_5$ alkylene radical, the reaction being conducted in the presence of a basic reagent.

**Claims for the following Contracting State : ES**

**1.** A process for the preparation of 3-sulphonyl indolizine derivatives of general formula:

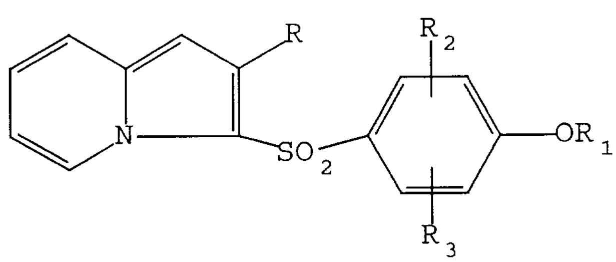

I

in which:

R represents hydrogen, a linear or branched $C_1$-$C_8$ alkyl radical, a $C_3$-$C_6$ cycloalkyl radical, an unsubstituted phenyl group or a phenyl group substituted by one or more substituents, identical or different, selected from halogen atoms, $C_1$-$C_4$ alkyl groups, $C_1$-$C_4$ alkoxy groups or the nitro group,

$R_1$ represents a protecting group for hydroxyl, namely a methyl, benzyl, $C_1$-$C_4$ alkylsulphonyl or $C_6$-$C_{10}$ arylsulphonyl group,

$R_2$ and $R_3$, which may be identical or different, each represents hydrogen, a methyl or ethyl group, or a

halogen atom;
characterised in that a 3-phenylthio indolizine derivative of general formula:

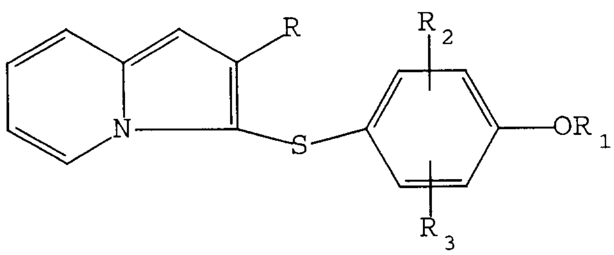

II

in which R, $R_1$, $R_2$ and $R_3$ are as defined previously, is oxidised at room temperature by means of 3-chloroperbenzoic acid in the presence of a basic reagent and in a $C_1$-$C_4$ lower alcohol as solvent.

**2.** A process according to claim 1, characterised in that the basic reagent is an alkali metal carbonate.

**3.** A process according to claim 1, characterised in that the solvent is methanol.

**4.** A process according to any of claims 1 to 3, characterised in that 1 to 5 equivalents of 3-chloroperbenzoic acid are used per equivalent of 3-phenylthio indolizine derivative of formula II.

**5.** A process according to claim 4, characterised in that 4 equivalents of 3-chloroperbenzoic acid are used.

**6.** A process according to one of claims 1 to 5, characterised in that 2 to 4 equivalents of basic reagent are used per equivalent of 3-chloroperbenzoic acid.

**7.** A process for the preparation of a 3-aminoalkoxyphenyl-sulphonyl indolizine derivative of general formula:

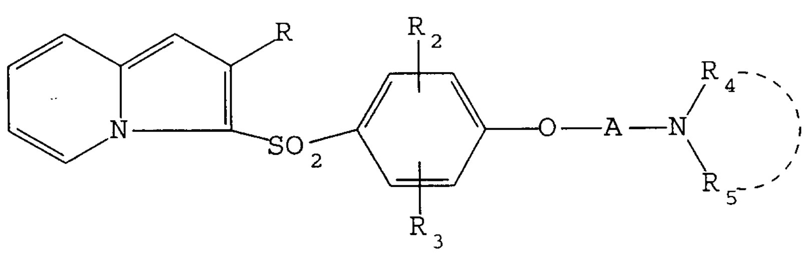

Ia

as well as its pharmaceutically acceptable salts, in which R, $R_2$ and $R_3$ are as defined in claim 1,
A represents a linear or branched $C_2$-$C_5$ alkylene radical, or a 2-hydroxy propylene radical in which the hydroxy is optionally substituted by a $C_1$-$C_4$ alkyl radical,
$R_4$ represents a linear or branched $C_1$-$C_8$ alkyl radical or a radical of formula:

- Alk-$R_6$

in which Alk represents a single bond or a linear or branched $C_1$-$C_5$ alkylene radical, and $R_6$ represents a pyridyl, phenyl, 2,3-methylenedioxyphenyl, or 3,4-methylenedioxyphenyl radical or a phenyl group substituted by one or more substituents, identical or different, selected from halogen atoms, $C_1$-$C_4$ alkyl groups or $C_1$-$C_4$ alkoxy groups,
$R_5$ represents hydrogen or a $C_1$-$C_8$ alkyl radical, or $R_4$ and $R_5$, when they are taken together, represent a $C_3$-$C_6$ alkylene or alkenylene radical, optionally substituted by a phenyl radical or optionally interrupted by -O-, -N= or -N(|)-$R_7$,
$R_7$ representing hydrogen, a $C_1$-$C_4$ alkyl group or a phenyl group, characterised in that

a) deprotection of the p-hydroxyphenylsulphonyl group of the compound of formula I as defined previously is carried out in order to obtain a p-hydroxyphenylsulphonyl derivative of general formula:

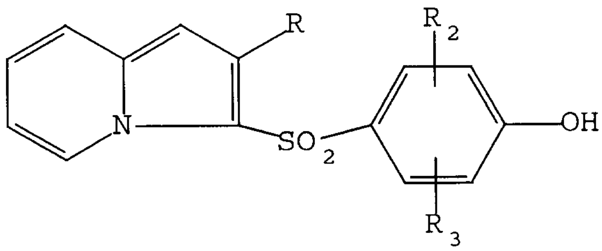

IV

in which R, $R_2$ and $R_3$ are as defined previously;

b) the condensation of the compound of formula IV is carried out with an alkane dihalide of general formula:

Hal-A-Hal V

in which A represents an alkylene radical as defined in formula Ia and Hal represents a halogen atom, under reflux in a solvent such as methyl ethyl ketone or N,N-dimethylformamide and in the presence of a basic reagent,

or alternatively

$b_1$) condensation with a halogenated alcohol of general formula:

Hal-A-OH VI

in which A represents an alkylene radical as defined in formula Ia and Hal is as defined previously, is carried out in a solvent such as N,N-dimethylformamide and in the presence of a basic reagent, followed by the condensation of the alcohol derivative obtained with a halide of general formula:

Hal-W VII

in which W represents a $C_1$-$C_4$ alkylsulphonyl or $C_6$-$C_{10}$ arylsulphonyl radical, in an acid-acceptor solvent,

or alternatively

$b_2$) heating under reflux with an epihalohydrin such as epichlorhydrin or epibromohydrin in the presence of a basic reagent and in a polar solvent

in order to obtain a 3-sulphonyl indolizine derivative of general formula:

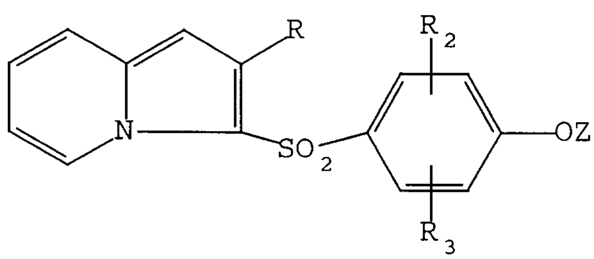

VIII

in which R, $R_2$ and $R_3$ are as defined previously and Z represents an oxiranylmethyl radical or a radical of formula:

$-A-Z_1$

in which A represents a linear or branched $C_2$-$C_5$ alkyleneradical, and $Z_1$ represents a halogen atom, a $C_1$-$C_4$ alkylsulphonyloxy radical, or a $C_6$-$C_{10}$ arylsulphonyloxy radical.

c) the derivative of formula VIII is reacted with an amine of general formula:

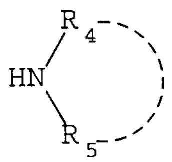

IX

in which $R_4$ and $R_5$ are as defined previously, the reaction taking place in the presence of an acid-acceptor in a suitable solvent in order to obtain a compound of formula Ia in the form of the free base, which may be reacted, if desired, with a suitable acid in order to form a pharmaceutically acceptable salt of this compound.

**8.** A process for the preparation of a 3-aminoalkoxyphenyl-sulphonyl indolizine derivative of general formula:

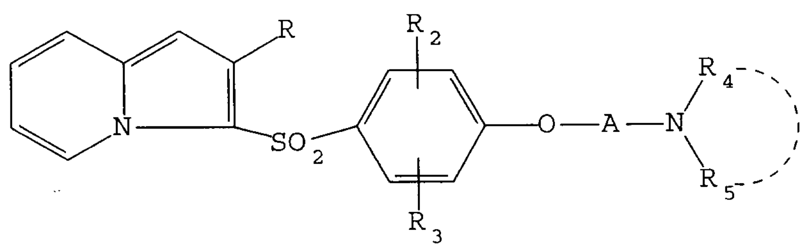

Ia

as well as its pharmaceutically acceptable salts, in which R, $R_2$ and $R_3$ are as defined in claim 1,
A represents a linear or branched $C_2$-$C_5$ alkylene radical,
$R_4$ represents a linear or branched $C_1$-$C_8$ alkyl radical or a radical of formula:

$-Alk-R_6$

in which
Alk represents a single bond or a linear or branched $C_1$-$C_5$ alkylene radical, and $R_6$ represents a pyridyl, phenyl, 2,3-methylenedioxyphenyl, or 3,4-methylenedioxyphenyl radical, or a phenyl group substituted by one or more substituents, identical or different, selected from halogen atoms, $C_1$-$C_4$ alkyl groups or $C_1$-$C_4$ alkoxy groups,
$R_5$ represents hydrogen or a $C_1$-$C_8$ alkyl radical or $R_4$ and $R_5$, when they are taken together, represent a $C_3$-$C_6$ alkylene or alkenylene radical optionally substituted by a phenyl radical or optionally interrupted by -O-, -N= or $-\overset{|}{N}-R_7$,
$R_7$ representing hydrogen, a $C_1$-$C_4$ alkyl radical or a phenyl radical, characterised in that

a) deprotection of the p-hydroxyphenylsulphonyl group of a compound of formula I as defined in claim 1 is carried out in order to obtain a p-hydroxyphenylsulphonyl derivative of general formula:

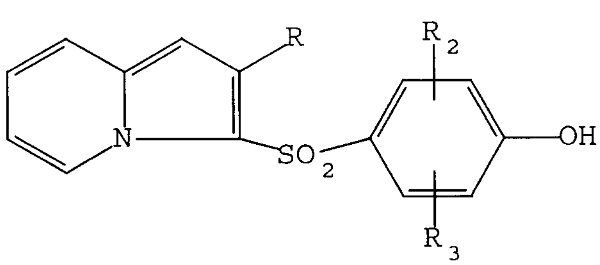

IV

in which R, $R_2$ and $R_3$ are as defined previously, and

b) the compound of formula IV is treated with a halide of general formula:

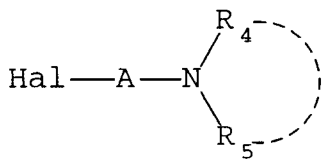

X

in which Hal, $R_4$ and $R_5$ are as defined previously and A represents a $C_2$-$C_5$ alkylene radical, the reaction being conducted in the presence of a basic reagent.

**9.** A process for the preparation of 3-sulphonyl indolizine derivatives according to claim 1, characterised in that a derivative of formula I is prepared in which R represents an isopropyl or cyclopropyl group.

**10.** A process for the preparation of 3-sulphonyl indolizine derivatives according to claim 1 or claim 9, characterised in that a derivative of formula I is prepared in which $R_1$ represents a methyl group.

**11.** A process for the preparation of 3-sulphonyl indolizine derivatives according to claim 9, characterised in that 2-isopropyl-3-p-methoxyphenylsulphonyl indolizine is prepared.

**Patentansprüche**
**Patentansprüche für folgende Vertragsstaaten : AT, BE, CH, DE, FR, GB, GR, IT, LI, LU, NL, SE**

**1.** 3-Sulfonyl-indolizin-Derivate der allgemeinen Formel:

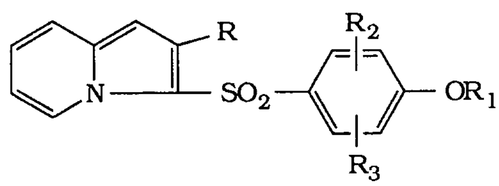

I

in der
R Wasserstoff, eine geradkettige oder verzweigte $C_1$-$C_8$-Alkylgruppe, eine $C_3$-$C_6$-Cycloalkylgruppe oder eine Phenylgruppe, die gegebenenfalls durch einen oder mehrere, gleichartige oder verschiedenartige Substituenten, ausgewählt aus Halogenatomen und $C_1$-$C_4$-Alkylgruppen, $C_1$-$C_4$-Alkoxygruppen und Nitrogruppen substituiert ist,
$R_1$ eine Schutzgruppe für eine Hydroxylgruppe, d. h. eine Methylgruppe, Benzylgruppe, $C_1$-$C_4$-Alkylsulfonylgruppe oder $C_6$-$C_{10}$-Arylsulfonylgruppe, und
$R_2$ und $R_3$, die gleichartig oder verschieden sein können, jeweils Wasserstoff, Methylgruppen, Ethylgruppen oder Halogenatome bedeuten.

**2.** 3-Sulfonyl-indolizin-Derivate nach Anspruch 1, worin R eine Isopropylgruppe oder eine Cyclopropylgruppe bedeutet.

**3.** 3-Sulfonyl-indolizin-Derivate nach Anspruch 1 oder 2, worin $R_1$ eine Methylgruppe darstellt.

**4.** 2-Isopropyl-3-p-methoxyphenylsulfonyl-indolizin.

**5.** Verfahren zur Herstellung der 3-Sulfonyl-indolizin-Derivate nach einem der Ansprüche 1 bis 4, **dadurch gekennzeichnet**, daß man ein 3-Phenylthio-indolizin-Derivat der allgemeinen Formel:

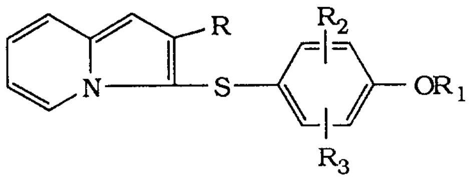

II

in der R, $R_1$, $R_2$ und $R_3$ die oben angegebenen Bedeutungen besitzen, mit 3-Chlor-perbenzoesäure in Gegenwart eines basischen Mittels und in einem niedrigmolekularen $C_1$-$C_4$-Alkohol als Lösungsmittel oxidiert.

**6.** Verfahren nach Anspruch 5, **dadurch gekennzeichnet**, daß das basische Mittel ein Alkalimetallcarbonat ist.

**7.** Verfahren nach Anspruch 5, **dadurch gekennzeichnet**, daß das Lösungsmittel Methanol ist.

**8.** Verfahren nach einem der Ansprüche 5 bis 7, **dadurch gekennzeichnet**, daß man 1 bis 5 Äquivalente der 3-Chlor-perbenzoesäure pro Äquivalent des 3-Phenylthio-indolizin-Derivats der Formel II verwendet.

**9.** Verfahren nach Anspruch 8, **dadurch gekennzeichnet**, daß man 4 Äquivalente der 3-Chlor-perbenzoesäure verwendet.

**10.** Verfahren nach einem der Ansprüche 5 bis 9, **dadurch gekennzeichnet**, daß man 2 bis 4 Äquivalente des basischen Mittels pro Äquivalent der 3-Chlor-perbenzoesäure verwendet.

**11.** Verfahren zur Herstellung eines 3-Aminoalkoxyphenylsulfonyl-indolizin-Derivats der allgemeinen Formel:

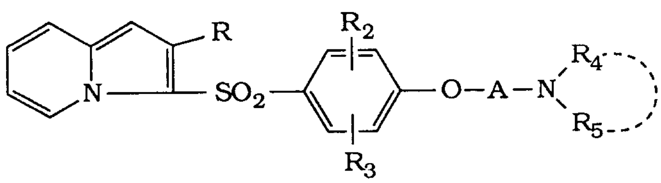

Ia

sowie von dessen pharmazeutisch annehmbaren Salzen, worin R, $R_2$ und $R_3$ die in Anspruch 1 angegebenen Bedeutungen besitzen,
A eine geradkettige oder verzweigte $C_2$-$C_5$-Alkylengruppe oder eine 2-Hydroxypropylengruppe, worin die Hydroxylgruppe gegebenenfalls durch eine $C_1$-$C_4$-Alkylgruppe substituiert ist,
$R_4$ eine geradkettige oder verzweigte $C_1$-$C_8$-Alkylgruppe oder eine Gruppe der Formel:

- Alk - $R_6$

worin Alk eine Einfachbindung oder eine geradkettige oder verzweigte $C_1$-$C_5$-Alkylengruppe und $R_6$ eine Pyridylgruppe, Phenylgruppe, 2,3-Methylendioxy-phenylgruppe, 3,4-Methylendioxy-phenylgruppe oder eine Phenylgruppe, die durch einen oder mehrere identische oder verschiedenartige Substituenten ausgewählt aus Halogenatomen, $C_1$-$C_4$-Alkylgruppen und $C_1$-$C_4$-Alkoxygruppen substituiert ist, darstellen.
$R_5$ ein Wasserstoffatom oder eine $C_1$-$C_8$-Alkylgruppe oder $R_4$ und $R_5$ gemeinsam eine $C_3$-$C_6$-Alkylengruppe oder $C_3$-$C_6$-Alkenylengruppe, die gegebenenfalls durch eine Phenylgruppe substituiert ist, oder gegebenenfalls durch - O-, -N= oder -N-$R_7$ unterbrochen ist, worin $R_7$ Wasserstoff, eine $C_1$-$C_4$-Alkylgruppe oder eine Phenylgruppe darstellt, bedeuten, **dadurch gekennzeichnet**, daß man

a) von einer Verbindung der Formel I, wie sie in Anspruch 1 definiert ist, die Schutzgruppe der p-Hydroxyphenylsulfonylgruppe abspaltet zur Bildung eines p-Hydroxyphenylsulfonyl-Derivats der allgemeinen Formel:

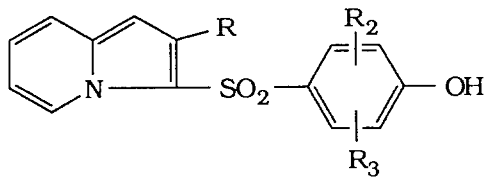

IV

in der R, $R_2$ und $R_3$ die oben angegebenen Bedeutungen besitzen,
b) die Verbindung der Formel IV am Rückfluß in einem Lösungsmittel, wie Methylethylketon oder N,N-Dimethylformamid und in Gegenwart eines basischen Mittels mit einem Dihalogenalkan der allgemeinen Formel:

Hal - A - Hal    V

in der A eine Alkylengruppe, wie sie oben bezüglich der Formel Ia definiert worden ist, und Hal ein Halogenatom bedeuten, kondensiert,
oder
$b_1$) mit einem halogenierten Alkohol der allgemeinen Formel:

Hal - A - OH    VI

in der A eine Alkylengruppe, wie sie bezüglich der Formel Ia definiert worden ist, darstellt und Hal die oben angegebenen Bedeutungen besitzt, in Gegenwart eines basischen Mittels kondensiert und dann das erhaltene Alkoholderivat mit einem Halogenid der allgemeinen Formel:

Hal - W    VII

In der W eine $C_1$-$C_4$-Alkylsulfonylgruppe oder eine $C_6$-$C_{10}$-Arylsulfonylgruppe darstellt, in einem als Säureakzeptor geeigneten Lösungsmittel kondensiert.
oder
$b_2$) am Rückfluß mit einem Epihalogenhydrin, wie Epichlorhydrin oder Epibromhydrin, in Gegenwart eines basischen Mittels und in einem polaren Lösungsmittel erhitzt zur Bildung eines 3-Sulfonyl-indolizin-Derivats der allgemeinen Formel:

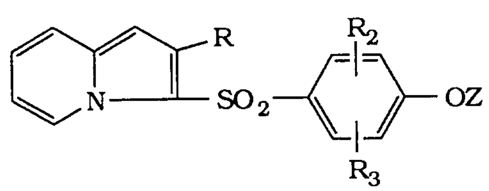

VIII

In der R, $R_2$ und $R_3$ die oben angegebenen Bedeutungen besitzen und Z eine Oxyranylmethylgruppe oder eine Gruppe der Formel:

- A - $Z_1$

worin A eine geradkettige oder verzweigte $C_2$-$C_5$-Alkylengruppe und $Z_1$ ein Halogenatom, eine $C_1$-$C_4$-Alkylsulfonyloxygruppe oder eine $C_6$-$C_{10}$-Arylsulfonyloxygruppe darstellen, erhält,
c) man das Derivat der Formel VIII mit einem Amin der allgemeinen Formel:

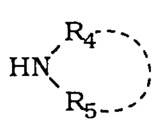

IX

In der $R_4$ und $R_5$ die oben angegebenen Bedeutungen besitzen. In Gegenwart eines Säureakzeptors und in einem geeigneten Lösungsmittel umsetzt zur Bildung einer Verbindung der Formel Ia in Form der freien Base, welche man gewünschtenfalls mit einer geeigneten Säure umsetzen kann zur Bildung eines pharmazeutisch annehmbaren Salzes dieser Verbindung.

**12.** Verfahren zur Herstellung eines 3-Aminoalkoxyphenylsulfonyl-indolizin-Derivats der allgemeinen Formel:

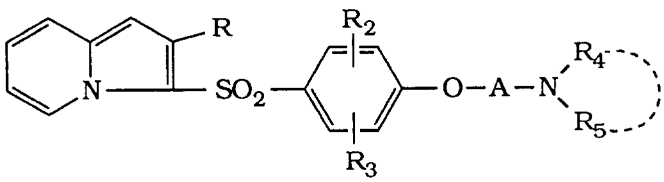

Ia

sowie von dessen pharmazeutisch annehmbaren Salzen, worin R, $R_2$ und $R_3$ die in Anspruch 1 angegebenen Bedeutungen besitzen,
A eine geradkettige oder verzweigte $C_2$-$C_5$-Alkylengruppe,
$R_4$ eine geradkettige oder verzweigte $C_1$-$C_8$-Alkylgruppe oder eine Gruppe der Formel:

- Alk - $R_6$

in der Alk eine Einfachbindung oder eine geradkettige oder verzweigte $C_1$-$C_5$-Alkylengruppe und $R_6$ eine Pyridylgruppe, eine Phenylgruppe, eine 2,3-Methylendioxy-phenylgruppe, eine 3,4-Methylendioxyphenylgruppe oder eine Phenylgruppe, die durch einen oder mehrere gleichartige oder verschiedene Substituenten ausgewählt aus Halogenatomen, $C_1$-$C_4$-Alkylgruppen und $C_1$-$C_4$-Alkoxygruppen substituiert ist, darstellen,
$R_5$ Wasserstoff oder eine $C_1$-$C_8$-Alkylgruppe oder $R_4$ und $R_5$ gemeinsam eine $C_3$-$C_6$-Alkylen- oder $C_3$-$C_6$-Alkenylengruppe, die gegebenenfalls durch eine Phenylgruppe substituiert oder gegebenenfalls durch -O-, -N= oder -N-$R_7$ unterbrochen ist, worin $R_7$ Wasserstoff, eine $C_1$-$C_4$-Alkylgruppe oder eine Phenylgruppe darstellt, bedeuten,
**dadurch gekennzeichnet**, daß man

a) von einer Verbindung der Formel I, wie sie in Anspruch 1 definiert ist, die Schutzgruppe der p-Hydroxyphenylsulfonylgruppe abspaltet, so daß man ein p-Hydroxyphenylsulfonyl-Derivat der allgemeinen Formel:

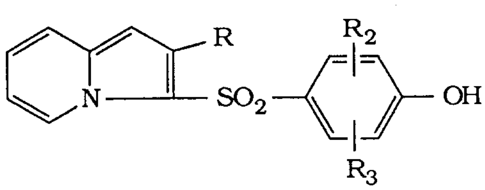

IV

erhält, in der R, $R_2$ und $R_3$ die oben angegebenen Bedeutungen besitzen, und
b) die Verbindung der Formel IV mit einem Halogenid der allgemeinen Formel:

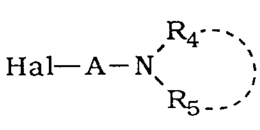

X

in der Hal, $R_4$ und $R_5$ die oben angegebenen Bedeutungen besitzen und A eine $C_2$-$C_5$-Alkylengruppe darstellt, in Gegenwart eines basischen Mittels behandelt.

**Patentansprüche für folgenden Vertragsstaat : ES**

1. Verfahren zur Herstellung von 3-Sulfonyl-indolizin-Derivaten der allgemeinen Formel:

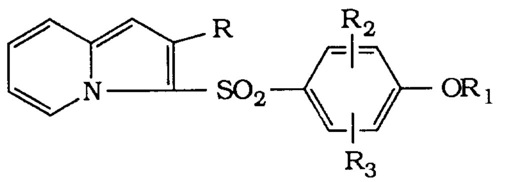

I

in der
R Wasserstoff, eine geradkettige oder verzweigte $C_1$-$C_8$-Alkylgruppe, eine $C_3$-$C_6$-Cycloalkylgruppe oder eine Phenylgruppe, die gegebenenfalls durch einen oder mehrere, gleichartige oder verschiedenartige Substituenten, ausgewählt aus Halogenatomen und $C_1$-$C_4$-Alkylgruppen, $C_1$-$C_4$-Alkoxygruppen und Nitrogruppen substituiert ist,
$R_1$ eine Schutzgruppe für eine Hydroxylgruppe, d. h. eine Methylgruppe, Benzylgruppe, $C_1$-$C_4$-Alkylsulfonylgruppe oder $C_6$-$C_{10}$-Arylsulfonylgruppe, und
$R_2$ und $R_3$, die gleichartig oder verschieden sein können, jeweils Wasserstoff, Methylgruppen, Ethylgruppen oder Halogenatome bedeuten, **dadurch gekennzeichnet**, daß man ein 3-Phenylthio-indolizin-Derivat der allgemeinen Formel:

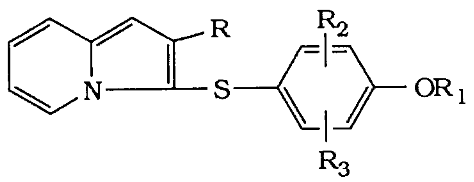

II

in der R, $R_1$, $R_2$ und $R_3$ die oben angegebenen Bedeutungen besitzen, mit 3-Chlor-perbenzoesäure In Gegenwart eines basischen Mittels und in einem niedrigmolekularen $C_1$-$C_4$-Alkohol als Lösungsmittel oxidiert.

2. Verfahren nach Anspruch 1, **dadurch gekennzeichnet**, daß das basische Mittel ein Alkalimetallcarbonat ist.

3. Verfahren nach Anspruch 1, **dadurch gekennzeichnet**, daß das Lösungsmittel Methanol ist.

4. Verfahren nach einem der Ansprüche 1 bis 3, **dadurch gekennzeichnet**, daß man 1 bis 5 Äquivalente 3-Chlor-perbenzoesäure pro Äquivalent des 3-Phenylthio-indolizin-Derivats der Formel II verwendet.

5. Verfahren nach Anspruch 4, **dadurch gekennzeichnet**, daß man 4 Äquivalente der 3-Chlor-perbenzoesäure verwendet.

6. Verfahren nach einem der Ansprüche 1 bis 5, **dadurch gekennzeichnet**, daß man 2 bis 4 Äquivalente des basischen Mittels pro Äquivalent der 3-Chlor-perbenzoesäure verwendet.

7. Verfahren zur Herstellung eines 3-Aminoalkoxyphenylsulfonyl-indolizin-Derivats der allgemeinen Formel:

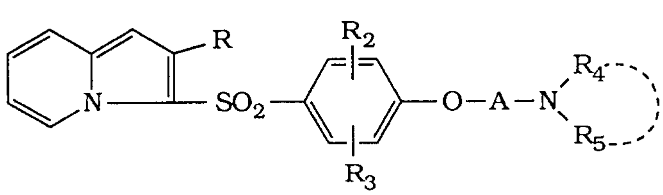

Ia

sowie von dessen pharmazeutisch annehmbaren Salzen, worin R, $R_2$ und $R_3$ die in Anspruch 1 angegebenen Bedeutungen besitzen,
A eine geradkettige oder verzweigte $C_2$-$C_5$-Alkylengruppe oder eine 2-Hydroxypropylengruppe, worin die Hydroxylgruppe gegebenenfalls durch eine $C_1$-$C_4$-Alkylgruppe substituiert ist,
$R_4$ eine geradkettige oder verzweigte $C_1$-$C_8$-Alkylgruppe oder eine Gruppe der Formel:

- Alk - $R_6$

worin Alk eine Einfachbindung oder eine geradkettige oder verzweigte $C_1$-$C_5$-Alkylengruppe und $R_6$ eine Pyridylgruppe, Phenylgruppe, 2,3-Methylendioxy-phenylgruppe, 3,4-Methylendioxy-phenylgruppe oder eine Phenylgruppe, die durch einen oder mehrere identische oder verschiedenartige Substituenten ausgewählt aus Halogenatomen, $C_1$-$C_4$-Alkylgruppen und $C_1$-$C_4$-Alkoxygruppen substituiert ist, darstellen,
$R_5$ ein Wasserstoffatom oder eine $C_1$-$C_8$-Alkylgruppe oder $R_4$ und $R_5$ gemeinsam eine $C_3$-$C_6$-Alkylengruppe oder $C_3$-$C_6$-Alkenylengruppe, die gegebenenfalls durch eine Phenylgruppe substituiert ist, oder gegebenenfalls durch - O-, -N= oder -N-$R_7$ unterbrochen ist, worin $R_7$ Wasserstoff, eine $C_1$-$C_4$-Alkylgruppe oder eine Phenylgruppe darstellt, bedeuten, **dadurch gekennzeichnet**, daß man

a) von einer Verbindung der Formel I, wie sie in Anspruch 1 definiert ist, die Schutzgruppe der p-Hydroxyphenylsulfonylgruppe abspaltet zur Bildung eines p-Hydroxyphenylsulfonyl-Derivats der allgemeinen Formel:

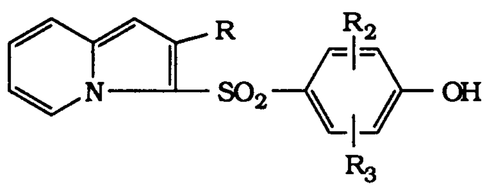

IV

in der R, $R_2$ und $R_3$ die oben angegebenen Bedeutungen besitzen,
b) die Verbindung der Formel IV am Rückfluß in einem Lösungsmittel, wie Methylethylketon oder N,N-Dimethylformamid und in Gegenwart eines basischen Mittels mit einem Dihalogenalkan der allgemeinen Formel:

Hal - A - Hal V

in der A eine Alkylengruppe, wie sie oben bezüglich der Formel Ia definiert worden ist, und Hal ein Halogenatom bedeuten, kondensiert,
<u>oder</u>

$b_1$) mit einem halogenierten Alkohol der allgemeinen Formel:

Hal - A - OH VI

in der A eine Alkylengruppe, wie sie bezüglich der Formel Ia definiert worden ist, darstellt und Hal die oben angegebenen Bedeutungen besitzt, in Gegenwart eines basischen Mittels kondensiert und dann das erhaltene Alkoholderivat mit einem Halogenid der allgemeinen Formel:

Hal - W VII

in der W eine $C_1$-$C_4$-Alkylsulfonylgruppe oder eine $C_6$-$C_{10}$-Arylsulfonylgruppe darstellt, in einem als Säureakzeptor geeigneten Lösungsmittel kondensiert,
<u>oder</u>
$b_2$) am Rückfluß mit einem Epihalogenhydrin, wie Epichlorhydrin oder Epibromhydrin, in Gegenwart eines basischen Mittels und in einem polaren Lösungsmittel erhitzt zur Bildung eines 3-Sulfonyl-indolizin-Derivats der allgemeinen Formel:

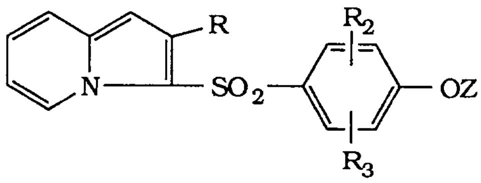

VIII

in der R, $R_2$ und $R_3$ die oben angegebenen Bedeutungen besitzen und Z eine Oxyranylmethylgruppe oder eine Gruppe der Formel:

- A - $Z_1$

worin A eine geradkettige oder verzweigte $C_2$-$C_5$-Alkylengruppe und $Z_1$ ein Halogenatom, eine $C_1$-$C_4$-Alkylsulfonyloxygruppe oder eine $C_6$-$C_{10}$-Arylsulfonyloxygruppe darstellen, erhält,

c) man das Derivat der Formel VIII mit einem Amin der allgemeinen Formel:

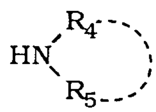

IX

in der $R_4$ und $R_5$ die oben angegebenen Bedeutungen besitzen, in Gegenwart eines Säureakzeptors und in einem geeigneten Lösungsmittel umsetzt zur Bildung einer Verbindung der Formel Ia in Form der freien Base, welche man gewünschtenfalls mit einer geeigneten Säure umsetzen kann zur Bildung eines pharmazeutisch annehmbaren Salzes dieser Verbindung.

**8.** Verfahren zur Herstellung eines 3-Aminoalkoxyphenylsulfonyl-indolizin-Derivats der allgemeinen Formel:

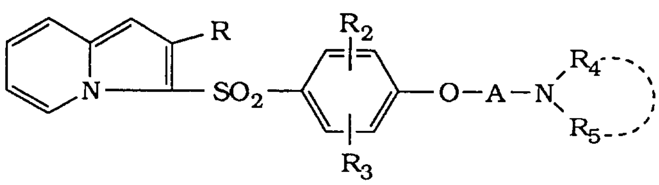

Ia

sowie von dessen pharmazeutisch annehmbaren Salzen, worin R, $R_2$ und $R_3$ die in Anspruch 1 angegebenen Bedeutungen besitzen,
A eine geradkettige oder verzweigte $C_2$-$C_5$-Alkylengruppe,
$R_4$ eine geradkettige oder verzweigte $C_1$-$C_8$-Alkylgruppe oder eine Gruppe der Formel:

- Alk - $R_6$

in der Alk eine Einfachbindung oder eine geradkettige oder verzweigte $C_1$-$C_5$-Alkylengruppe und $R_6$ eine Pyridylgruppe, eine Phenylgruppe, eine 2,3-Methylendioxy-phenylgruppe, eine 3,4-Methylendioxyphenylgruppe oder eine Phenylgruppe, die durch einen oder mehrere gleichartige oder verschiedene Substituenten ausgewählt aus Halogenatomen, $C_1$-$C_4$-Alkylgruppen und $C_1$-$C_4$-Alkoxygruppen substituiert ist, darstellen,
$R_5$ Wasserstoff oder eine $C_1$-$C_8$-Alkylgruppe oder $R_4$ und $R_5$ gemeinsam eine $C_3$-$C_6$-Alkylen- oder $C_3$-$C_6$-Alkenylengruppe, die gegebenenfalls durch eine Phenylgruppe substituiert oder gegebenenfalls durch -O-, -N= oder -N-$R_7$ unterbrochen ist, worin $R_7$ Wasserstoff, eine $C_1$-$C_4$-Alkylgruppe oder eine Phenylgruppe darstellt, bedeuten,
**dadurch gekennzeichnet**, daß man

a) von einer Verbindung der Formel I, wie sie in Anspruch 1 definiert ist, die Schutzgruppe der p-Hydroxyphenylsulfonylgruppe abspaltet, so daß man ein p-Hydroxyphenylsulfonyl-Derivat der allgemeinen Formel:

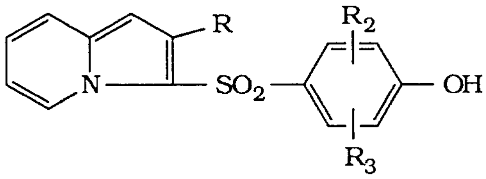

IV

erhält, in der R, $R_2$ und $R_3$ die oben angegebenen Bedeutungen besitzen, und
b) die Verbindung der Formel IV mit einem Halogenid der allgemeinen Formel:

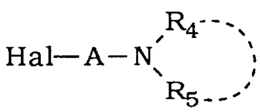

X

in der Hal, $R_4$ und $R_5$ die oben angegebenen Bedeutungen besitzen und A eine $C_2$-$C_5$-Alkylengruppe darstellt, in Gegenwart eines basischen Mittels behandelt.

**9.** Verfahren zur Herstellung der 3-Sulfonyl-indolizin-Derivate nach Anspruch 1, **dadurch gekennzeichnet**, daß man ein Derivat der Formel I herstellt, in der R eine Isopropylgruppe oder eine Cyclopropylgruppe bedeutet.

**10.** Verfahren zur Herstellung von 3-Sulfonyl-indolizin-Derivate nach Anspruch 1 oder Anspruch 9, **dadurch gekennzeichnet**, daß man ein Derivat der Formel I herstellt, in der $R_1$ eine Methylgruppe bedeutet.

**11.** Verfahren zur Herstellung von 3-Sulfonyl-indolizin-Derivaten nach Anspruch 9, **dadurch gekennzeichnet**, daß man 2-Isopropyl-3-p-methoxyphenylsulfonyl-indolizin herstellt.